# EUROPÄISCHE PATENTSCHRIFT

(11) **EP 0 601 386 B1**
(45) Veröffentlichungstag und Bekanntmachung des Hinweises auf die Patenterteilung: **05.02.2003**
(21) Anmeldenummer: 93118869.2
(22) Anmeldetag: 24.11.1993
(51) Int. Cl.: C07D 239/46, A61K 31/505

(54) **Neue Sulfonamide**
Sulphonamides
Sulfonamides

(30) Priorität: 10.12.1992 CH 377792; 11.12.1992 CH 379992; 14.10.1993 CH 311493
(43) Veröffentlichungstag der Anmeldung: 15.06.1994
(73) Patentinhaber: F. HOFFMANN-LA ROCHE AG, 4002 Basel (CH)
(72) Erfinder: Breu, Volker, D-79418 Schliengen (DE); Burri, Kaspar, CH-4102 Binningen (CH); Cassal, Jean-Marie, F-68100 Mulhouse (FR); Clozel, Martine, F-68300 St. Louis (FR); Hirth, Georges, F-68330 Huningue (FR); Löffler, Bernd-Michael, D-79206 Oberrimsingen (DE); Müller, Marcel, CH-4402 Frenkendorf (CH); Neidhart, Werner, F-68870 Bartenheim (FR); Ramuz, Henri, CH-4127 Birsfelden (CH)
(74) Vertreter: Witte, Hubert, Dr.

(56) Entgegenhaltungen:
- EP-A- 0 510 526
- CHEMICAL ABSTRACTS, vol. 108, no. 11, 14. März 1988, Columbus, Ohio, US; abstract no. 94000y, GRUENDEMANN E ET AL. 'Carbon-13 nmr studies on 4-amino-2,6-dialkylpyrimidines and their sulfonamide derivatives' Seite 606 ;Spalte 2 ; & J. PRAKT. CHEM., Bd.329, Nr.3, 1987 Seiten 391 - 399
- CHEMICAL ABSTRACTS, vol. 104, no. 12, 24. März 1986, Columbus, Ohio, US; abstract no. 95587z, MUCH H. ET AL. 'HPLC-studies of sulfonamides:....' Seite 455 ;Spalte 2 ; & PHARMAZIE, Bd.40, Nr.9, 1985 Seiten 627 - 628

## Beschreibung

Die vorliegende Erfindung betrifft neue Sulfonamide und deren Verwendung als Heilmittel. Insbesondere betrifft die Erfindung neue Verbindungen der Formel 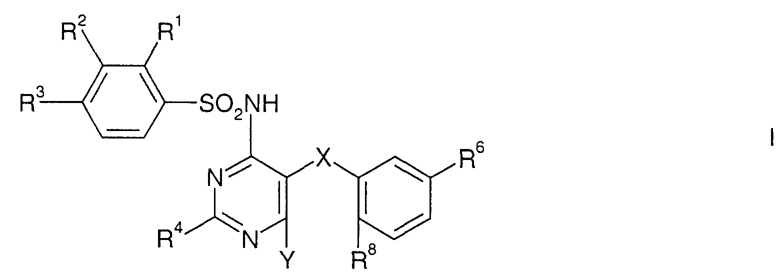 worin
- R¹: Wasserstoff, C₁₋₇-Alkyl, C₁₋₇-Alkoxy, C₁₋₇-Alkylthio, Halogen oder Trifluormethyl;
- R²: Wasserstoff, C₁₋₇-Alkyl, C₁₋₇-Alkoxy, Halogen, Trifluormethyl oder -OCH₂COOR⁹;
- R³: Wasserstoff, C₁₋₇-Alkyl; C₁₋₇-Alkoxy, Halogen, C₁₋₇-Alkylthio, Trifluormethyl oder Trifluormethoxy;
- R² und R³: zusammen Butadienyl, Methylendioxy, Aethylendioxy oder Isopropylidendioxy;
- R⁴: Wasserstoff, 2-Pyrimidinyl, 2- und 3-Furyl, 2- und 3-Thienyl, p-Methoxyphenyl oder Morpholino;
- R⁶: C₁₋₇-Alkoxy;
- R⁸: Halogen;
- X: -O- oder -S-;
- Y: -CHO, C₁₋₄-Alkyl, -(CH₂)₁₋₄-O-R⁹, -(CH₂)₁₋₄NHC(O)R¹⁰, -CH₂OCH₂CH(OH)CH₂OH und cycl. Ketale davon, -CH₂NR⁹CH₂CH(OH)CH₂OH, -CH₂OCH₂CH₂S(O)CH₃, -CH₂O(CH₂)₁₋₄-OH, -CH₂O(CH₂)₁₋₄OC(O)R¹⁰, -CH₂O(CH₂)₁₋₄OC(O)Het, oder -CH₂Hal;
- R⁹: Wasserstoff oder C₁₋₄-Alkyl;
- R¹⁰: C₁₋₄-Alkyl;
- Het oder Heterocyclyl: einen mono- oder bicyclischen 5- oder 6-gliedrigen Heterocyclus darstellt, der unsubstituiert oder mit Hal, C₁₋₇-Alkyl, C₁₋₇-Alkoxy, Aryl oder Aryl-C₁₋₇-Alkyl mono- oder di-substituiert sein kann und Sauerstoff, Stickstoff oder Schwefel als Heteroatom enthält;
- Aryl: Phenyl oder mit Halogen, C₁₋₇-Alkyl, C₁₋₇-Alkoxy, Carboxyl und/oder Trifluormethyl substituiertes Phenyl darstellt und
- Hal: Halogen ist;
und Salze davon.

Der hier verwendete Ausdruck "nieder" bezeichnet Gruppen mit 1-7 C-Atomen, vorzugsweise 1-4 C-Atomen. Alkyl-, Alkoxy- und Alkylthiogruppen sowie Alkylgruppen als Bestandteile von Alkanoylgruppen können geradkettig oder verzweigt sein. Beispiele solcher Alkylgruppen sind Methyl, Aethyl, Propyl, Isopropyl, Butyl, sek. und tert.Butyl. Halogen bezeichnet Fluor, Chlor, Brom und Jod, wobei Chlor bevorzugt ist. Beispiele von Arylresten sind Phenyl und substituierte Phenylreste, wobei als Substituenten insb. Halogen, nieder-Alkyl, nieder-Alkoxy, Carboxyl und Trifluormethyl in Betracht kommen. Beispiele von Heterocyclylresten sind substituierte, z.B. durch nieder-Alkyl, nieder-Alkoxy, Halogen, Aryl, Aryl-nieder-alkyl mono- oder disubstituierte oder unsubstituierte mono- oder bicyclische 5- und 6-gliedrige heterocyclische Reste mit Sauerstoff, Stickstoff oder Schwefel als Heteroatom, wie 2- und 3-Furyl, 2-, 4- und 5-Pyrimidinyl, 2-, 3- und 4-Pyridyl und Pyridyl-N-oxid, 1,2- und 1,4-Diazinyl, Morpholino, 2- und 3-Thienyl, Isoxazolyl, Oxazolyl, Thiazolyl, Imidazolyl, Pyrrolyl, Benzofuranyl, Benzothienyl, Indolyl, Purinyl, Chinolyl, Isochinolyl und Chinazolyl. Beispiele von Salzen sind Alkalisalze, wie Na- und K-Salze, und Erdalkalimetallsalze, wie Ca- und Mg-Salze.

Die Verbindungen der oben angegebenen Formel I sind Hemmstoffe für Endothelin-Rezeptoren. Sie können daher zur Behandlung von Erkrankungen, die mit Endothelin-Aktivitäten assoziiert sind, insbesondere Kreislauferkrankungen, wie Hypertonie, Ischämie, Vasospasmen und Angina pectoris verwendet werden.

EP-A-0 510 526 offenbart Sulfonamid-Verbindungen als Hemmstoffe für Endothelin-Rezeptoren. Überraschend wurde nun gefunden, dass die Sulfonamide der vorliegenden Erfindung eine unerwartet hohe antagonistische Potenz in vitro aufweisen.

Eine bevorzugte Gruppe von Verbindungen innerhalb der Formel I sind diejenigen, worin X -O- ist und weiterhin diejenigen, in denen R⁶ Methoxy und R⁸ Chlor bedeuten.

Bevorzugte Substituenten R¹ und R² sind Wasserstoff, bevorzugte Substituenten R³ nieder-Alkyl, besonders tert.Butyl. Bevorzugter Substituent R⁴ ist Morpholino.

Bevorzugte Substituenten Y sind -CHO, C₁₋₄-Alkyl, -(CH₂)₁₋₄-O-R⁹, -(CH₂)₁₋₄NHC(O)R¹⁰, -CH₂OCH₂CH(OH)CH₂OH und cycl. Ketale davon, -CH₂NR⁹CH₂CH(OH)CH₂OH, -CH₂OCH₂CH₂S(O)CH₃, -CH₂O(CH₂)₁₋₄-OH, -CH₂O(CH₂)₁₋₄OC(O)R¹⁰, -CH₂O(CH₂)₁₋₄OC(O)Het, -CH₂Hal, insbesondere Hydroxymethyl, 2-Hydroxy-ethoxymethyl und 2,3-Dihydroxypropoxymethyl.

Die Verbindungen der Formel I können dadurch hergestellt werden, dass man
a) eine Verbindung der Formel 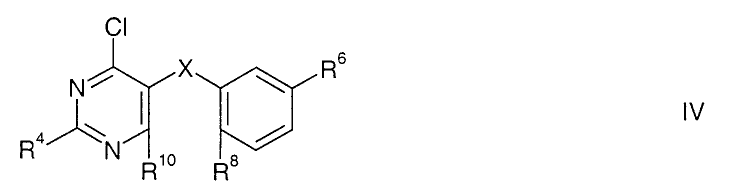 worin R⁴-R⁸, R¹⁰ und X die obige Bedeutungen haben,
   mit einer Verbindung der Formel 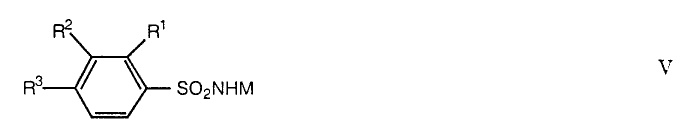 worin M ein Kation darstellt,
   zu einer Verbindung der Formel I, worin Y ein Rest R¹⁰ ist und R¹⁻⁸ und X die obige Bedeutung haben, umsetzt, gegebenenfalls die erhaltene Verbindung der Formel I, in der Y ein Rest CH₃ ist, zu einer Verbindung der Formel I, in der Y ein Rest CHO ist, oxidiert und gegebenenfalls den Rest CHO in einen anderen, oben definierten Rest Y überführt, oder

Die Umsetzung der Verbindungen IV mit den Verbindungen V kann in für die Herstellung von Sulfonamiden an sich bekannter Weise vorgenommen werden, z.B. in einem inerten organischen Lösungsmittel, wie Dimethylsulfoxid, zweckmässig unter Erwärmen und in einer Schutzgasatmosphäre, z.B. unter Argon. In den Verbindungen der Formel V ist das Kation vorzugsweise ein Alkalimetallkation, wie Na⁺ oder K⁺.

Die wie vorstehend beschrieben erhaltenen Verbindungen der Formel I, in denen Y ein Rest CH₃, z.B. Methyl, ist, können durch Abwandlung von Substituenten in andere Verbindungen der Formel I übergeführt werden. So kann die den Substituenten Y repräsentierende Gruppe CH₃ durch Oxidation in eine Gruppe CHO umgewandelt werden. Die Oxidation kann in an sich bekannter Weise, z.B. mit Selendioxid, vorgenommen werden. In der so erhaltenen Verbindung kann die Formylgruppe zur Hydroxymethylgruppe reduziert werden. Diese Reduktion kann in an sich bekannter Weise, z.B. mittels Reduktionsmitteln wie NaBH₄, bewerkstelligt werden. Die Hydroxymethyl- (bzw. alkyl)gruppe kann durch Umsetzung mit einem Halogenierungsmittel, wie POCl₃/PCl₅ in ein Halogenmethyl- (bzw. alkyl)gruppe übergeführt werden, aus denen durch Umsetzung mit Alkoholen oder Aminoalkoholen Verbindungen der Formel I erhalten werden, in denen Y ein Rest -(CH₂)₁₋₄OR⁹, -CH₂OCH₂CH(OH)CH₂OH und cyclische Ketale davon, -CH₂NR⁹CH₂CH(OH)CH₂OH oder -CH₂O(CH₂)₁₋₄OH darstellt. In den so erhaltenen Verbindungen der Formel I können in Y enthaltene Hydroxy- oder Aminogruppen verestert werden, wobei Verbindungen der Formel I erhalten werden, in denen Y einen der oben aufgeführten Reste darstellt, in denen der Rest R¹¹ in der Bedeutung C₁₋₄-Alkanoyl oder Heterocyclylcarbonyl enthalten ist. Andererseits kann die Formylgruppe in einer Grignard-Reaktion mit Alkylmagnesiumhalogeniden in eine Verbindung der Formel I umgewandelt werden, in der Y ein Rest -CH(OH)R¹¹ ist. Die Formylgruppe kann weiterhin mit Grignard-Verbindungen der Formel BrMg-(CH₂)₁₋₄-Z-H umgesetzt werden, in denen OH- und SH-Gruppen in geschützter Formel (z.B. als Benzyläther) vorliegen, um (nach Abspaltung von OH- und SH-Schutzgruppen) Verbindungen der Formel I mit Y = -CH(OH)(CH₂)₁₋₄-O-H zu erhalten. Durch Umsetzung einer Verbindung der Formel I mit Dimethylsulfid/Li können Verbindungen der Formel I mit Y = -CH(OH)CH₂SCH₃ erhalten werden, die mit NaJO₄ zu Verbindungen der Formel I mit Y = -CH(OH)CH₂S(O)CH₃ oxidiert werden können.

Verbindungen der Formel I mit Y = -C(O)R¹⁰ oder -C(O)(CH₂)₁₋₄-O-R¹¹ können durch Oxidation entsprechender Verbindungen, in denen Y ein Rest -CH(OH)R¹⁰ oder -CH(OH)(CH₂)₁₋₄OR¹¹ ist, hergestellt werden, wobei OH-Gruppen OR¹¹ zweckmässig intermediär geschützt werden, z.B. als Benzyläther. Als Oxidationsmittel für diese Oxidation kommt z.B. CrO₃/Pyridin in Betracht.

Alle diese Umsetzungen können auf an sich bekannte Weise vorgenommen werden. Schliesslich können die Verbindungen der Formel I in an sich bekannter Weise in Salze, z.B. Alkalisalze wie Na- und K-Salze überführt werden.

Die als Ausgangsmaterial eingesetzten Verbindungen können, soweit sie nicht bekannt sind oder ihre Herstellung nachstehend beschrieben ist, in Analogie zu bekannten bzw. den nachstehend beschriebenen Methoden hergestellt werden.

Verbindungen der Formel IV mit n = 1 können wie folgt hergestellt werden:

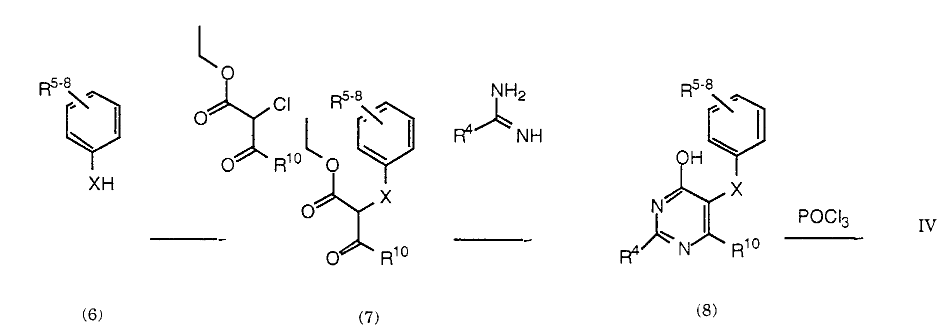

Ein Phenol oder Thiophenol der Formel (6) kann in Gegenwart von Natrium in einem geeigneten Lösungsmittel, z.B. Toluol, mit 2-Chloracetessigsäureäthylester zu einer Verbindung der Formel (7) umgesetzt werden, aus der durch Kondensation mit dem Amidin R⁴C(NH)NH₂ das Hydroxy-Pyrimidinderivat (8) bzw. sein Tautomer -NH-CO- hergestellt werden kann. Austausch der Hydroxygruppe gegen Chlor mittels POCl₃ liefert die Verbindung IV.

Die Hemmwirkung der Verbindungen der Formel I auf Endothelinrezeptoren kann mit der nachstehend beschriebenen Versuchsanordnungen gezeigt werden.

### I. Hemmung der Endothelin-Bindung an Human-Placentamembranen (vgl. Life Sci 44:1429 (1989))

Humanplacenta wird in 5 mM Tris-Puffer, pH 7.4, der 1 mM MgCl₂ und 250 mM Sucrose enthält, homogenisiert. Das Homogenisat wird mit 3000 g 15 Minuten bei 4°C zentrifugiert, der die Plasmamembranfraktion enthaltende Ueberstand mit 72000 g 30 Minuten zentrifugiert und der Bodenkörper mit 75 mM Tris-Puffer, pH 7.4, der 25 mM MgCl₂ enthält, gewaschen. Danach wird der aus jeweils 10 g Originalgewebe erhaltene Bodenkörper in 1 ml 75 mM Tris-Puffer pH 7.4, enthaltend 25 mM MgCl₂ und 250 mM Sucrose, suspendiert und in 1 ml Aliquots bei -20°C eingefroren.

Für den Bindungs-Assay werden die eingefrorenen Membranpräparate aufgetaut und nach 10 Minuten Zentrifugieren mit 25000 g bei 20°C in Assay-Puffer (50 mM Tris-Puffer pH 7.4, enthaltend 25 mM MnCl₂, 1 mM EDTA und 0,5% Rinderserumalbumin) resuspendiert. 100 µl dieser Membransuspension, enthaltend 70 µg Protein werden mit 50 µl ¹²⁵I-Endothelin (spezif. Aktivität 2200 Ci/mMol) in Assay-Puffer (25000 cpm, Endkonzentration 20 pM) und 100 µl Assay-Puffer, der variierende Konzentrationen der Testverbindung enthält, inkubiert. Die Inkubation wird 2 Stunden bei 20°C oder 24 Stunden bei 4°C durchgeführt. Die Trennung von freiem und Membran-gebundenen Radioliganden wird durch Filtration über Glasfaserfilter vorgenommen.

### II. Hemmung Endothelin-induzierter Kontraktionen an isolierten Aortenringen der Ratte

Aus der Thoraxaorta von erwachsenen Wistar-Kyoto-Ratten wurden Ringe von 5 mm Länge herausgeschnitten. Das Endothel wird durch leichtes Reiben der Innenfläche entfernt. Jeder Ring wird bei 37°C in 10 ml Krebs-Henseleit-Lösung unter Begasung mit 95% O₂ und 5% CO₂ in ein isoliertes Bad eingetaucht. Die isometrische Spannung der Ringe wird gemessen. Die Ringe werden auf eine Vorspannung von 3 g gedehnt. Nach 10 Minuten Inkubation mit der Testverbindung oder Vehikel werden kumulative Dosen von Endothelin-1 zugegeben. Die Aktivität der Testverbindung wird durch Berechnung des Dosisverhältnisses, d.h. der durch 100 µM Testverbindung induzierten Rechtsverschiebung (Verschiebung zu höheren Werten) der EC₅₀ von Endothelin bestimmt, wobei EC₅₀ die für eine halbmaximale Kontraktion erforderliche Endothelin-Konzentration bezeichnet. Je grösser dieses Dosisverhältnis ist, desto potenter hemmt die Testverbindung die biologische Wirkung von Endothelin-1. Die EC₅₀ von Endothelin in Abwesenheit von Testverbindungen ist 0,3 nM.

### III. Die Hemmwirkung auf die Vasokonstriktion an der Ratte

Ratten werden mit Na-Thiobutabarbital (100 mg/kg i.p.) anästhesiert. Ueber die Femoralarterie wird ein Katheter zur Messung des systemischen arteriellen Blutdrucks und über die Femoralvene ein Katheter in die Vena cava inferior zur Injektion der Testverbindungen gelegt. Eine Dopplersonde wird um die linke Nierenarterie gelegt und mit einem Doppler-Messgerät verbunden. Durch 45 Minuten langes Abklemmen der linken Nierenarterie am Austrittsort wird eine renale Ischämie erzeugt. Die Testverbindungen werden 10 Minuten vor der Einleitung der Ischämie intraarteriell (i.a.) in Dosen von 5 mg/kg oder intravenös (i.v.) in Dosen von 10 mg/kg verabreicht. In Kontrollversuchen war die renale Durchblutung im Vergleich zum prä-ischämischen Wert um 43±4% vermindert.

In der Tabelle 1 ist die in der Versuchsanordnung I ermittelte Hemmwirkung von Verbindungen der Formel I als IC₅₀ angegeben, d.h. als Konzentration [µM] die erforderlich ist, 50% der spezifischen Bindung von ¹²⁵I-Endothelin zu hemmen.

**Tabelle 1**

| Verbindung von Beispiel | IC₅₀ [µM] |
|---|---|
| 3 | 0,130 |
| 5 | 0,035 |
| 10 | 0,184 |
| 13 | 0,182 |

Die Verbindungen der Formel I können aufgrund ihrer Fähigkeit, die Endothelinbindung zu hemmen, als Mittel zur Behandlung von Erkrankungen verwendet werden, die mit die Vasokonstriktion erhöhenden Vorgängen assoziiert sind. Beispiele solcher Erkrankungen sind Bluthochdruck, Koronarerkrankungen, Herzinsuffizienz, renale und myocardiale Ischämie, Niereninsuffizienz, Dialyse, cerebrale Ischämie, Hirninfarkt, Migräne, subarachnoidale Hämorrhagie, Raynaud-Syndrom und pulmonärer Hochdruck. Sie können ebenfalls bei Atherosklerose, Verhinderung der Restenosierung nach Ballon-induzierter Gefässdilatation, Entzündungen, Magen- und Duodenalulcera, Ulcus cruris, Gram-negativer Sepsis, Schock, Glomerulonephritis, Nierenkolik, Glaukom, Asthma, bei der Therapie und Prophylaxe diabetischer Komplikationen und Komplikationen bei der Verabreichung von Cyclosporin, sowie anderen, mit Endothelin-Aktivitäten assoziierten Erkrankungen Anwendung finden.

Die Verbindungen der Formel I können oral, rectal, parenteral, z.B. intravenös, intramuskulär, subcutan, intrathecal oder transdermal; oder sublingual oder als ophthalmologische Zubereitung, oder als Aerosol verabreicht werden. Beispiele von Applikationsformen sind Kapseln, Tabletten, oral verabreichbare Suspensionen oder Lösungen, Suppositorien, Injektionslösungen, Augentropfen, Salben oder Spraylösungen.

Eine bevorzugte Anwendungsform ist die intravenöse, intramuskuläre oder orale Applikation. Die Dosierung, in denen die Verbindungen der Formel I in wirksamen Mengen verabreicht werden, hängen von der Art des spezifischen Wirkstoffs, dem Alter und den Bedürfnissen des Patienten und der Applikationsweise ab. Im allgemeinen kommen Dosierungen von etwa 0,1-100 mg/kg Körpergewicht pro Tag in Betracht. Die die Verbindungen der Formel I enthaltenden Präparate können inerte oder auch pharmakodnymisch aktive Zusätze enthalten. Tabletten oder Granulate z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze, sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dergleichen bestehen. Voraussetzung ist, dass alle bei der Herstellung der Präparate verwendeten Hilfsstoffe untoxisch sind.

Die nachstehenden Beispiele erläutern die Erfindung weiter. Von den darin verwendeten Abkürzungen bedeutet Sdp. Siedepunkt; und Smp. Schmelzpunkt, MS Massenspektrum und M Molmasse.

### Beispiel 1

10.7 g 4- [4-Chloro-5-(2-chloro-5-methoxy-phenoxy)-6-methyl-pyrimidin-2-yl]-morpholin und 21.6 g p-t-Butyl-benzolsulfonamid-Kalium in 150 ml trockenem Dimethylsulfoxid wurden unter Argon 16 Stunden auf 120°C erwärmt. Danach wurde das Dimethylsulfoxid abdestilliert, der Rückstand zwischen Aethylacetat und IN Salzsäure verteilt und die organische Phase neutral gewaschen. Die organische Phase wurde getrocknet, das Lösungsmittel abgedampft und der Rückstand aus Dichlormethan-Aethanol umkristallisiert. Man erhielt 14.7 g 4-tert-Butyl-N-[5-(2-chloro-5-methoxyphenoxy)-6-methyl-2-(morpholin-4-yl)-pyrimidin-4-yl]-benzolsulfonamid, Smp 154°C, MS: M= 546.

Das Ausgangsmaterial wurde wie folgt hergestellt:
a) Zu einer Lösung von 17.1 g 2-Chlor-5-methoxyphenol in Toluol, wurde 2.8 g Natrium gegeben. Das Reaktionsgemisch wurde bei 110°C, unter Argon 3 Stunden gerührt, und danach mit einer Lösung von 19.57 g 2-Chloracetessigsäureaethylester in Toluol versetzt. Das Reaktionsgemisch wurde 3 Stunden bei 110°C weiter gerührt, zwischen Essigsäure-Wasser 20% und Toluol verteilt. Die organische Phase wurde getrocknet und das Lösungsmittel abdestilliert. Der Rückstand wurde über Kieselgel mit Dichlormethan gereinigt. Man erhielt 18.2 g (RS)-(2-Chloro-5-methoxy-phenoxy)-acetessigsäureaethylester als gelbliches Oel. MS: M= 286.
b) Zu einer Natriummethylatlösung aus 10 ml Methanol und 0.19 g Natrium wurde 0.8 g Morpholinoformamidin hydrobromid und 1 g (RS)-(2-Chloro-5-methoxy-phenoxy)-acetessigsäureaethylester gegeben. Das Reaktionsgemisch wurde 16 Stunden bei 80°C gerührt, auf pH 6 gestellt, und eingeengt. Der Rückstand wird zwischen Chloroform und Wasser verteilt. Nach Trocknen und Eindampfen des Lösungsmittels wurde der Rückstand aus Aethanol-Dichlormethan kristallisiert. Man erhält 0.45 g 5-(2-Chloro-5-methoxy-phenoxy)-6-methyl-2-(morpholin-4-yl)-pyrimidin-4-ol, Smp. 252°C, MS: M= 351.
c) 1.72 g 5-(2-Chloro-5-methoxy-phenoxy)-6-methyl-2-(morpholin-4-yl)-pyrimidin-4-ol wurden in 3.3 ml POCl₃ versetzt. Das Reaktionsgemisch wurde bei 120°C 2 Stunden gerührt, danach das überschüssige Reagens abdestilliert. Der Rückstand wurde in Chloroform aufgenommen und mit Wasser, 1N NaOH, und Wasser gewaschen. Die organische Phase wurde getrocknet, eingeengt und der Rückstand aus Aether umkristallisiert. Man erhielt 1.48 g 4-[4-Chloro-5-(2-chloro-5-methoxy-phenoxy)-6-methyl-pyrimidin-2-yl]-morpholin, Smp. 134°C, MS: M= 369.

### Beispiel 2

14.6 g 4-tert-Butyl-N-[5-(2-chloro-5-methoxy-phenoxy)-6-methyl-2-(morpholin-4-yl)-pyrimidin-4-yl]-benzolsulfonamid und 15.9 g Seleniumdioxid in 500 ml Dioxan wurden in einem Autoklav bei 170°C während 6 Stunden gerührt. Das Reaktionsgemisch wurde filtriert und das Filtrat eingeengt. Der Rückstand wurde zwischen Chloroform und Wasser verteilt. Die organische Phase wurde getrocknet, das Lösungsmittel abgedampft und der Rückstand aus Dichlormethan-Aethanol umkristallisiert. Man erhielt 10.3 g 4-tert-Butyl-N-[5-(2-chloro-5-methoxy-phenoxy)-6-formyl-2-(morpholin-4-yl)-pyrimidin-4-yl]-benzolsulfonamid, Smp. 235-236°C, MS: M= 561.

### Beispiel 3

7 g 4-tert-Butyl-N-[5-(2-chloro-5-methoxy-phenoxy)-6-formyl-2-(morpholin-4-yl)-pyrimidin-4-yl]-benzolsulfonamid in 300 ml Aethanol wurden mit 0.9 g Natriumborhydrid versetzt. Das Reaktionsgemisch wurde bei 80°C 1 Stunde gerührt. Danach wurde das Aethanol abdestilliert und der Rückstand zwischen Chloroform und IN HCl verteilt. Die organische Phase wurde mit Wasser gewaschen und getrocknet, das Lösungsmittel abgedampft und der Rückstand mit Dichlormethan über Kieselgel chromatographiert. Nach Umkristallisation aus Dichlormethan-Aethanol erhielt man 4.6 g 4-tert-Butyl-N- [5-(2-chloro-5-methoxy-phenoxy)-6-hydroxymethyl-2-(morpholin-4-yl)-pyrimidin-4-yl]-benzolsulfonamid, Smp. 103°C, MS: M= 563.

### Beispiel 4

4.57 g 4-tert-Butyl-N-[5-(2-chloro-5-methoxy-phenoxy)-6-hydroxymethyl-2-(morpholin-4-yl)-pyrimidin-4-yl]-benzolsulfonamid in 50 ml POCl₃ wurden mit 2.03 g PCl₅ während 2 Stunden bei 20°C gerührt. Danach wurde das POCl₃ abdestilliert und der Rückstand zwischen Essigester und wässrige 1 N NaOH verteilt. Die organische Phase wurde mit Wasser gewaschen, getrocknet und das Lösungsmittel abgedampft. Der Rückstand wurde über Kieselgel mit Dichlormethan und Chloroform chromatographiert, danach aus Dichlormethan-Aethanol umkristallisiert. Man erhielt 3.01 g 4-tert-Butyl-N-[5-(2-chloro-5-methoxy-phenoxy)-6-chloromethyl-2-(morpholin-4-yl)-pyrimidin-4-yl]-benzolsulfonamid, Smp.170°C, MS: M= 581.

### Beispiel 5

Zu einer Natriumglykolatlösung aus 2,5 g Aethylenglykol und 0.12 g Natrium wurden 1 g 4-tert-Butyl-N-[5-(2-chloro-5-methoxy-phenoxy)-6-chloromethyl-2-(morpholin-4-yl)pyrimidin-4-yl]-benzolsulfonamid gegeben. Das Reaktionsgemisch wurde unter Argon 72 Stunden bei 80°C gerührt. Danach wurde das Aethylenglykol abdestilliert und der Rückstand zwischen Aethylacetat und 1N Salzsäure verteilt. Die organische Phase wurde mit Wasser gewaschen, über Natriumsulfat getrocknet und das Lösungsmittel abdestilliert. Der Rückstand wurde über Kieselgel mit Aether chromatographiert. Man erhielt 0.85 g 4-tert-Butyl-N-[5-(2-chloro-5methoxy-phenoxy)-6-(2-hydroxy-ethoxymethyl)-2-(morpholin-4-yl)-pyrimidin-4-yl]-benzolsulfonamid. Smp 162-164°C, MS: M= 606.

### Beispiel 6

In Analogie zu Beispiel 1 wurde aus 4-Chloro-5-(2-chloro-5-methoxyphenoxy)-6-methyl-pyrimidin das 4-tert-Butyl-N- [5-(2-chloro-5-methoxyphenoxy)-6-methyl-pyrimidin-4-yl]-benzolsulfonamid erhalten. Smp. 191°C, MS: (M-Cl•)= 426.

Das Ausgangsmaterial wurde wie folgt hergestellt:

In Analogie zu Beispiel 1, Absatz a) wurde aus (RS)-(2-Chloro-5-methoxyphenoxy)-acetessigsäureaethylester und Formamidinacetat das 5-[2-Chloro-5-methoxy-phenoxy]-6-methyl-pyrimidin-4-ol als Wachs erhalten, MS: M= 266, welches in Analogie zu Beispiel 1, Absatz b) mit POCl₃ umgesetzt wurde.

### Beispiel 7

In Analogie zu Beispiel 2 wurde die in Beispiel 6 erhaltene Verbindung in das 4-tert-Butyl-N-[5-(2-chloro-5-methoxy-phenoxy)-6-formyl-pyrimidin-4-yl]-benzolsulfonamid. Smp. 99-101°C, MS: M= 475 übergeführt.

### Beispiel 8

In Analogie zu Beispiel 3 wurde aus der in Beispiel 7 erhaltenen Verbindung das 4-tert-Butyl-N-[5-(2-chloro-5-methoxy-phenoxy)6-hydroxy-methylpyrimidin-4-yl]-benzolsulfonamid erhalten, Smp. 188°C, MS: M= 477.

### Beispiel 9

In Analogie zu Beispiel 4 wurde aus der in Beispiel 8 hergestellten Verbindung das 4-tert-Butyl-N-[6-chloromethyl-5-(2-chloro-5-methoxyphenoxy)-pyrimidin-4-yl]-benzolsulfonamid. Smp. 170°C, MS: (M-Cl•)= 460 erhalten.

### Beispiel 10

In Analogie zu Beispiel 5 wurde aus 4-tert-Butyl-N-[6-chloromethyl-5-(2-chloro-5-methoxy-phenoxy)-pyrimidin-4-yl]-benzolsulfonamid das 4-tert-Butyl-N-[5-(2-chloro-5-methoxy-phenoxy)-6-(2-hydroxy-ethoxy-methyl)-pyrimidin-4-yl]-benzolsulfonamid erhalten. Smp. 80°C, MS: (M+H)⁺= 522.

### Beispiel 11

100 mg4-tert-Butyl-N-[5-(2-chloro-5-methoxy-phenoxy)-6-(2-hydroxyethoxy-methyl)-2-(morpholin-4-yl)-pyrimidin-4-yl]-benzolsulfonamid wurden mit 3-Thiophencarbonsäure unter folgenden Bedingungen verestert: man löste 100 mg des Sulfonamides, 175 mg N-Aethyl-N'-(3-dimethylaminopropyl)-carbodiimid-hydrochlorid, 150 mg Triaethylamin und 5 mg Dimethylaminopyridin in 10 ml Dichlormethan und liess die Lösung 2 Stunden bei Raumtemperatur stehen. Anschliessend wurde zur Trockne eingedampft. Der Rückstand wurde mit Toluol azeotropiert und anschliessend zwischen Essigester und 1 N HCl verteilt, dann mit Wasser gewaschen und wie üblich isoliert. Die Verbindung wurde über Kieselgel gereinigt mit Chloroform als Fliessmittel. Man erhielt 90 mg Thiophen-3-carbonsäure 2-[6-(4-tert-Butyl-phenylsulfonamino)-5-(2-chloro-5-methoxy-phenoxy)-2-(morpholin-4-yl)-pyrimidin-4-yl-methoxy]-ethylester, amorphes Pulver, MS: M= 716.

### Beispiel 12

In Analogie zu Beispiel 5 wurde aus 200 mg 4-tert-Butyl-N-[5-(2-chloro-5-methoxy-phenoxy)-6-chloromethyl-2-(morpholin-4-yl)-pyrimidin-4-yl]-benzolsulfonamid und (RS)-2,2-Dimethyl-1,3-dioxolan-4-methanol-Na das (RS)-4-tert-Butyl-N-[5-(2-chloro-5-methoxy-phenoxy)-6-(2,2-dimethyl-1,3-dioxolan-4-ylmethoxymethyl)-2-morpholin-4-yl)-pyrimidin-4-yl]-benzolsulfonamid erhalten.Smp. 155-156°C, MS: M= 676.

### Beispiel 13

Eine Lösung von 100 mg der in Beispiel 12 hergestellten Verbindung in 2 ml Dioxan wurde mit 1.5 ml IN HCl versetzt und 15 Minuten auf 80°C erwärmt. Nach dem Eindampfen wurde der Rückstand über Kieselgel mit Aether als Fliessmittel chromatographiert und lieferte 85 mg (R,S)-4-tert-Butyl-N-[5-(2-chloro-5-methoxy-phenoxy)-6-(2,3-dihydroxy-propoxymethyl)-2-(morpholin-4-yl)-pyrimidin-4-yl]-benzolsulfonamid als Schaum, MS: (M+H)⁺= 637.

### Beispiel 14

Durch Umsetzung von 4-tert-Butyl-N-[6-chloromethyl-5-(2-chloro-5-methoxy-phenoxy)-pyrimidin-4-yl]-benzolsulfonamid mit Propandiol-Na wurde das 4-tert-Butyl-N-[5-(2-chloro-5-methoxy-phenoxy)-6-(3-hydroxypropoxymethyl)-pyrimidin-4-yl]-benzolsulfonamid erhalten. MS: M-(Cl• + HC(O)CH₂CH₂OH) = 436.

### Beispiel 15

Durch Umsetzung von 4-tert-Butyl-N-[6-chloromethyl-5-(2-chloro-5-methoxy-phenoxy)-pyrimidin-4-yl]-benzolsulfonamid mit 1-Aminopropandiol wurde das (RS)-4-tert-Butyl-N-[5-(2-chloro-5-methoxy-phenoxy)-6-(2,3-dihydroxypropylaminomethyl)-pyrimidin-4-yl]-benzolsulfonamid erhalten. MS: (M+H)⁺ = 551.

### Beispiel 16

In Analogie zu Beispiel 12 wurde aus 4-tert-Butyl-N-[6-chloromethyl-5-(2-chloro-5-methoxy-phenoxy)-pyrimidin-4-yl]-benzolsulfonamid das (RS)-4-tert-Butyl-N-[5-(2-chloro-5-methoxy-phenoxy)-6-(2,2-dimethyl-1,3-dioxolan-4-ylmethoxymethyl)-pyrimidin-4-yl]-benzolsulfonamid erhalten. MS: (M+H)⁺ = 592.

### Beispiel 17

In Analogie zu Beispiel 13 wurde aus (RS)-4-tert-Butyl-N-[5-(2-chloro-5-methoxy-phenoxy)-6-(2,2-dimethyl-1,3-dioxolan-4-ylmethoxymethyl)-pyrimidin-4-yl]-benzolsulfonamid das (RS)-4-tert-Butyl-N-[5-(2-chloro-5-methoxy-phenoxy)-6-(2,3-dihydroxy-propoxymethyl)-pyrimidin-4-yl]-benzolsulfonamid erhalten. MS: M = 551.

### Beispiel 18

Durch Umsetzung von 4-tert-Butyl-N-[5-(2-chloro-5-methoxy-phenoxy)-6-(2-hydroxy-ethoxy-methyl)-pyrimidin-4-yl]-benzolsulfonamid mit Nicotinsäure wurde der Pyridin-3-ylessigsäure 2-[6-(4-tert-butyl-phenylsulfonylamino)-5-(2-chloro-5-methoxy-phenoxy)-pyrimidin-4-ylmethoxy]-ethylester erhalten. MS: M = 626.

### Beispiel 29

Durch Umsetzung von 4-tert-Butyl-N-[5-(2-chloro-5-methoxy-phenoxy)-6-(2-hydroxy-ethoxy-methyl)-pyrimidin-4-yl]-benzolsulfonamid mit Isonicotinsäure wurde der Pyridin-4-ylessigsäure 2-[6-(4-tert-butyl-phenylsulfonylamino)-5-(2-chloro-5-methoxy-phenoxy)-pyrimidin-4-ylmethoxy]-ethylester erhalten.

### Beispiel 20

Durch Umsetzung von 4-tert-Butyl-N-[5-(2-chloro-5-methoxy-phenoxy)-6-(2-hydroxy-ethoxy-methyl)-pyrimidin-4-yl]-benzolsulfonamid mit 3-Furancarbonsäure wurde der Furan-3-carbonsäure 2-[6-(4-tert-butyl-phenylsulfonylamino)-5-(2-chloro-5-methoxy-phenoxy)-pyrimidin-4-ylmethoxy]-ethylester erhalten. MS: (M+H)⁺ = 618.

### Beispiel 21

Durch Umsetzung von 4-tert-Butyl-N-[5-(2-chloro-5-methoxy-phenoxy)-6-(2-hydroxy-ethoxy-methyl)-pyrimidin-4-yl]-benzolsulfonamid mit 3-Thiophencarbonsäure wurde der Thiophen-3-carbonsäure 2-[6-(4-tert-butyl-phenylsulfonylamino)-5-(2-chloro-5-methoxy-phenoxy)-pyrimidin-4-ylmethoxy]-ethylester erhalten. MS: M = 631.

### Beispiel 22

In Analogie zu Beispiel 1 wurde aus 4-Chloro-5-(2-chloro-5-methoxyphenoxy)-6-methyl-2,2'-bipyrimidinyl das 4-tert-Butyl-N-[5-(2-chloro-5-methoxy-phenoxy)-6-methyl-2,2'-bipyrimidinyl-4-yl]-benzolsulfonamid erhalten, Smp. 122°C, MS: (M-Cl) = 504.

Das Ausgangsmaterial wurde wie folgt hergestellt:
(a) In Analogie zu Beispiel 1b) wurde aus 2-Pyrimidinoamidin hydrochlorid das 5-(2-Chloro-5-methoxy-phenoxy)-6-methyl-2,2'-bipyrimidinyl-4-ol erhalten. MS: M = 344.
(b) In Analogie zu Beispiel 1c) wurde aus der oben beschriebene Substanz das 4-Chloro-5-(2-chloro-5-methoxy-phenoxy)-6-methyl-2,2'-bipyrimidinyl erhalten, Smp. 110°C, MS: M = 363.

### Beispiel 23

In Analogie zu Beispiel 12 wurde das (S)-4-tert.-Butyl-N-[5-(2-chloro-5-methoxy-phenoxy)-6-(2,2-dimethyl-1,3-dioxolan-4-ylmethoxymethyl)-2-morpholin-4-yl)-pyrimidin-4-yl]-benzolsulfonamid erhalten. MS: M = 677

### Beispiel 24

In Analogie zu Beispiel 12 wurde das (R)-4-tert.-Butyl-N-[5-(2-chloro-5-methoxy-phenoxy)-6-(2,2-dimethyl-1,3-dioxolan-4-ylmethoxymethyl)-2-(morpholin-4-yl)-pyrimidin-4-yl]-benzolsulfonamid erhalten. MS: M = 677.

### Beispiel 25

In Analogie zu Beispiel 13 wurde aus der in Beispiel 24 hergestellten Verbindung das (S)-4-tert.-Butyl-N-[5-(2-chloro-4-methoxy-phenoxy)-6-(2,3-dihydroxypropoxymethyl)-2-(morpholin-4-yl)-pyrimidin-4-yl]-benzolsulfonamid erhalten. MS: M = 637.

### Beispiel 26

In Analogie zu Beispiel 13 wurde aus der in Beispiel 24 hergestellten Verbindung das (R)-4-tert.-Butyl-N-[5-(2-chloro-5-methoxy-phenoxy)-6-(2,3-dihydroxy-propoxymethyl)-2-(morpholin-4-yl)-pyrimidin-4-yl-benzolsulfonamid erhalten. MS: M = 637.

### Beispiel 27

In Analogie zu Beispiel 16 wurde aus 4-tert.-Butyl-N-[6-chlormethyl-5-(2-chloro-5-methoxy-phenoxy)-pyrimidin-4-yl]benzolsulfonamid und 2,3-O-Isopropyliden-L-threit das (4S,5S)-4-tert.-Butyl-N-[5-(2-chloro-5-methoxyphenoxy)-6-(5-hydroxymethyl-2,2-dimethyl-1,3-dioxolan-4-ylmethoxymethyl)-pyrimidin-4-yl]-benzolsulfonamid erhalten. MS: M = 622.

### Beispiel 28

In Analogie zu Beispiel 2 wurde aus den in Beispiel 22 hergestellten Verbindung der 6-(4-tert.-Butyl-phenylsulfonamino)-5-(2-chloro-5-methoxyphenoxy)-2,2'-bipyrimidinyl-4-carboxaldehyd erhalten, Smp. 211°C.

### Beispiel 29

In Analogie zu Beispiel 3 wurde aus den in Beispiel 28 hergestellten Aldehyde das 4-tert.-Butyl-N-[5-(2-chloro-5-methoxy-phenoxy)-6-hydroxymethyl-2,2'-bipyrimidin-4-yl]-benzolsulfonamid erhalten, MS: M = 556.

### Beispiel 30

In Analogie zu Beispiel 4 wurde aus dem in Beispiel 29 hergestellten Alkohol das 4-tert.-Butyl-N-[5-(2-chloro-5-methoxy-phenoxy)-6-chloromethyl-2,2'-bipyrimidin-4-yl]-benzolsulfonamid erhalten, MS: M = 574.

### Beispiel 31

In Analogie zu Beispiel 5 wurde aus den in Beispiel 30 hergestellten Substanz das 4-tert.-Butyl-N-[5-(2-chloro-5-methoxy-phenoxy)-6-(2-hydroxyethoxymethyl)-2-2'-bipyrimidin-4-yl]-benzolsulfonamid erhalten, MS: (M-H)⁻ = 598.

### Beispiel A

Tabletten enthaltend die folgenden Bestandteile können in herkömmlicher Weise hergestellt werden:

| Bestandteile | pro Tablette |
|---|---|
| Verbindung der Formel I | 10,0 - 100,0 mg |
| Lactose | 125,0 mg |
| Maisstärke | 75,0 mg |
| Talk 4,0 mg | |
| Magnesiumstearat | 1,0 mg |

### Beispiel B

Kapseln enthaltend die folgenden Bestandteile können in herkömmlicher Weise hergestellt werden:

| Bestandteile | pro Kapsel |
|---|---|
| Verbindung der Formel I | 25,0 mg |
| Lactose | 150,0 mg |
| Maisstärke | 20,0 mg |
| Talk 5,0 mg | |

### Beispiel C

Injektionslösungen können folgende Zusammensetzung aufweisen:

| | |
|---|---|
| Verbindung der Formel I | 3,0 mg |
| Gelatine | 150,0 mg |
| Phenol | 4,7 mg |
| Wasser für Injektionslösungen | ad 1,0 ml |

### Beispiel D

In 3,5 ml Myglyol 812 und 0,08 g Benzylalkohol werden 500 mg Verbindung der Formel I suspendiert. Diese Suspension wird in einen Behälter mit Dosierventil eingefüllt. Es werden 5,0 g Freon 12 unter Druck durch das Ventil in den Behälter abgefüllt. Durch Schütteln wird das Freon in der Myglyol-Benzylalkoholmischung gelöst. Dieser Spraybehälter enthält ca. 100 Einzeldosen, die einzeln appliziert werden können.

## Patentansprüche

1. Verbindungen der Formel 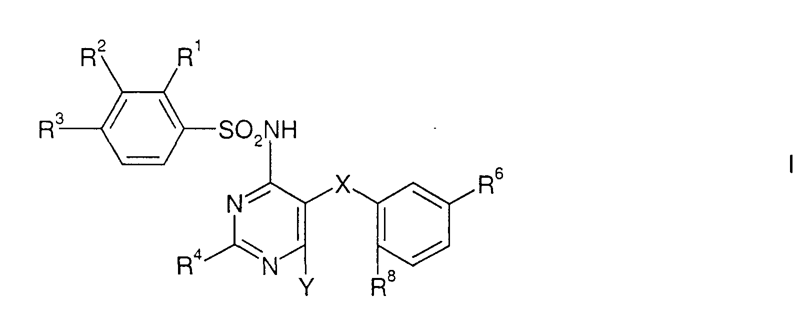 worin
R¹ Wasserstoff, C₁₋₇-Alkyl, C₁₋₇-Alkoxy, C₁₋₇-Alkylthio, Halogen oder Trifluormethyl;
R² Wasserstoff, C₁₋₇-Alkyl, C₁₋₇-Alkoxy, Halogen, Trifluormethyl oder - OCH₂COOR⁹;
R³ Wasserstoff, C₁₋₇-Alkyl; C₁₋₇-Alkoxy, Halogen, C₁₋₇-Alkylthio, Trifluormethyl oder Trifluormethoxy;
R² und R³ zusammen Butadienyl, Methylendioxy, Aethylendioxy oder Isopropylidendioxy;
R⁴ Wasserstoff, 2-Pyrimidinyl, 2- und 3-Furyl, 2- und 3-Thienyl, p-Methoxyphenyl oder Morpholino;
R⁶ C₁₋₇-Alkoxy;
R⁸ Halogen;
X -O- oder -S-;
Y -CHO, C₁₋₄-Alkyl, -(CH₂)₁₋₄-O-R⁹, -(CH₂)₁₋₄NHC(O)R¹⁰, -CH₂OCH₂CH(OH)CH₂OH und cycl. Ketale davon, -CH₂NR⁹CH₂CH(OH)CH₂OH, -CH₂OCH₂CH₂S(O)CH₃, -CH₂O(CH₂)₁₋₄-OH, -CH₂O(CH₂)₁₋₄OC(O)R¹⁰, -CH₂O(CH₂)₁₋₄OC(O)Het, oder -CH₂Hal;
R⁹ Wasserstoff oder C₁₋₄-Alkyl;
R¹⁰ C₁₋₄-Alkyl;
Het oder Heterocyclyl einen mono- oder bicyclischen 5- oder 6-gliedrigen Heterocyclus darstellt, der unsubstituiert oder mit Hal, C₁₋₇-Alkyl, C₁₋₇-Alkoxy, Aryl oder Aryl-C₁₋₇-Alkyl mono- oder di-substituiert sein kann und Sauerstoff, Stickstoff oder Schwefel als Heteroatom enthält;
Aryl Phenyl oder mit Halogen, C₁₋₇-Alkyl, C₁₋₇-Alkoxy, Carboxyl und/oder Trifluormethyl substituiertes Phenyl darstellt und
Hal Halogen ist;
und Salze davon.

2. Verbindungen gemäss Anspruch 1, worin Y Hydroxymethyl, 2-Hydroxy-ethoxymethyl oder 2,3-Dihydroxypropoxymethyl bedeutet.

3. Verbindungen gemäss den Ansprüchen 1-2, worin R¹ und R² Wasserstoff und R³ C₁₋₇-Alkyl bedeuten.

4. Die Verbindungen gemäss Anspruch 1,
4-tert-Butyl-N-[5-(2-chloro-5-methoxy-phenoxy)-6-methyl-2-(morpholin-4-yl)-pyrimidin-4-yl]-benzolsulfonamid,
4-tert-Butyl-N-[5-(2-chloro-5-methoxy-phenoxy)-6-formyl-2-(morpholin-4-yl)-pyrimidin-4-yl]-benzolsulfonamid,
4-tert-Butyl-N-[5-(2-chloro-5-methoxy-phenoxy)-6-hydroxymethyl-2-(morpholin-4-yl)-pyrimidin-4-yl]-benzolsulfonamid,
4-tert-Butyl-N-[5-(2-chloro-5-methoxy-phenoxy)-6-chloromethyl-2-(morpholin-4-yl)-pyrimidin-4-yl]-benzolsulfonamid,
4-tert-Butyl-N-[5-(2-chloro-5methoxy-phenoxy)-6-(2-hydroxy-ethoxymethyl)-2-(morpholin-4-yl)-pyrimidin-4-yl]-benzolsulfonamid,
4-tert-Butyl-N-[5-(2-chloro-5-methoxy-phenoxy)-6-methyl-pyrimidin-4-yl]-benzolsulfonamid,
4-tert-Butyl-N-[5-(2-chloro-5-methoxy-phenoxy)-6-formyl-pyrimidin-4-yl]-benzolsulfonamid,
4-tert-Butyl-N-[5-(2-chloro-5-methoxy-phenoxy)6-hydroxy-methyl-pyrimidin-4-yl] -benzolsulfonamid,
4-tert-Butyl-N-[6-chloromethyl-5-(2-chloro-5-methoxy-phenoxy)-pyrimidin-4-yl] -benzolsulfonamid,
4-tert-Butyl-N-[5-(2-chloro-5-methoxy-phenoxy)-6-(2-hydroxy-ethoxy-methyl)-pyrimidin-4-yl]-benzolsulfonamid,
Thiophen-3-carbonsäure 2-[6-(4-tert-Butyl-phenylsulfonamino)-5-(2-chloro-5-methoxy-phenoxy)-2-(morpholin-4-yl)-pyrimidin-4-yl-methoxy]-ethylester,
(RS)-4-tert-Butyl-N-[5-(2-chloro-5-methoxy-phenoxy)-6-(2,2-dimethyl-1,3-dioxolan-4-ylmethoxymethyl)-2-morpholin-4-yl)-pyrimidin-4-yl]-benzolsulfonamid,
(R,S)-4-tert-Butyl-N-[5-(2-chloro-5-methoxy-phenoxy)-6-(2,3-dihydroxypropoxymethyl)-2-(morpholin-4-yl)-pyrimidin-4-yl]-benzolsulfonamid,
4-tert-Butyl-N-[5-(2-chloro-5-methoxy-phenoxy)-6-(3-hydroxy-propoxymethyl)-pyrimidin-4-yl]-benzolsulfonamid,
(RS)-4-tert-Butyl-N-[5-(2-chloro-5-methoxy-phenoxy)-6-(2,3-dihydroxypropylaminomethyl)-pyrimidin-4-yl]-benzolsulfonamid,
(RS)-4-tert-Butyl-N- [5-(2-chloro-5-methoxy-phenoxy)-6-(2,2-dimethyl-1,3-dioxolan-4-ylmethoxymethyl)-pyrimidin-4-yl]-benzolsulfonamid,
(RS)-4-tert-Butyl-N- [5-(2-chloro-5-methoxy-phenoxy)-6-(2,3-dihydroxypropoxymethyl)-pyrimidin-4-yl]-benzolsulfonamid,
Pyridin-3-ylessigsäure 2-[6-(4-tert-butyl-phenylsulfonylamino)-5-(2-chloro-5-methoxy-phenoxy)-pyrimidin-4-ylmethoxy]-ethylester,
Pyridin-4-ylessigsäure 2-[6-(4-tert-butyl-phenylsulfonylamino)-5-(2-chloro-5-methoxy-phenoxy)-pyrimidin-4-ylmethoxy]-ethylester,
Furan-3-carbonsäure 2-[6-(4-tert-butyl-phenylsulfonylamino)-5-(2-chloro-5-methoxy-phenoxy)-pyrimidin-4-ylmethoxy]-ethylester,
Thiophen-3-carbonsäure 2-[6-(4-tert-butyl-phenylsulfonylamino)-5-(2-chloro-5-methoxy-phenoxy)-pyrimidin-4-ylmethoxy]-ethylester und
4-tert-Butyl-N-[5-(2-chloro-5-methoxy-phenoxy)-6-methyl-2,2'-bipyrimidinyl-4-yl]-benzolsulfonamid,
(S)-4-tert.-Butyl-N-[5-(2-chloro-5-methoxy-phenoxy)-6-(2,2-dimethyl-1,3-dioxolan-4-ylmethoxymethyl)-2-morpholin-4-yl)-pyrimidin-4-yl]-benzolsulfonamid,
(R)-4-tert.-Butyl-N- [5-(2-chloro-5-methoxy-phenoxy)-6-(2,2-dimethyl-1,3-dioxolan-4-ylmethoxymethyl)-2-(morpholin-4-yl)-pyrimidin-4-yl]-benzolsulfonamid,
(S)-4-tert.-Butyl-N-[5-(2-chloro-4-methoxy-phenoxy)-6-(2,3-dihydroxypropoxymethyl)-2-(morpholin-4-yl)-pyrimidin-4-yl]-benzolsulfonamid,
(R)-4-tert.-Butyl-N-[5-(2-chloro-5-methoxy-phenoxy)-6-(2,3-dihydroxypropoxymethyl)-2-(morpholin-4-yl)-pyrimidin-4-yl-benzolsulfonamid,
6-(4-tert.-Butyl-phenylsulfonamino)-5-(2-chloro-5-methoxy-phenoxy)-2,2'bipyrimidinyl-4-carboxaldehyd,
4-tert.-Butyl-N-[5-(2-chloro-5-methoxy-phenoxy)-6-hydroxymethyl-2,2'bipyrimidin-4-yl]-benzolsulfonamid,
4-tert.-Butyl-N-[5-(2-chloro-5-methoxy-phenoxy)-6-chloromethyl-2,2'bipyrimidin-4-yl]-benzolsulfonamid and
4-tert.-Butyl-N- [5-(2-chloro-5-methoxy-phenoxy)-6-(2-hydroxy-ethoxymethyl)-2-2'-bipyrimidin-4-yl]-benzolsulfonamid.

5. Die Verbindungen gemäss den Ansprüchen 1-4 zur Anwendung als Heilmittel.

6. Verwendung der Verbindungen der Ansprüche 1-4 bei der Herstellung von Heilmitteln zur Behandlung von Erkrankungen, die mit Endothelin-Aktivitäten assoziiert sind, insbesondere Kreislauferkrankungen wie Hypertonie, Ischämie, Vasospasmen und Angina pectoris.

7. Pharmazeutische Präparate in Dosiseinheitsform enthaltend eine Verbindung der Ansprüche 1-4 und einen pharmazeutisch üblichen Trägerstoff.

8. Verfahren zur Herstellung der Verbindungen der Ansprüche 1-4, **dadurch gekennzeichnet, dass** man eine Verbindung der Formel 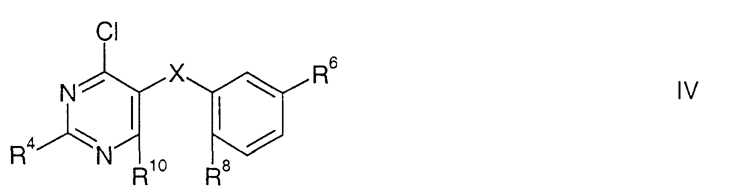 worin R⁴-R⁸ und X die in Anspruch 1 genannten Bedeutungen haben und
R¹⁰ C₁₋₄-Alkyl ist,
mit einer Verbindung der Formel 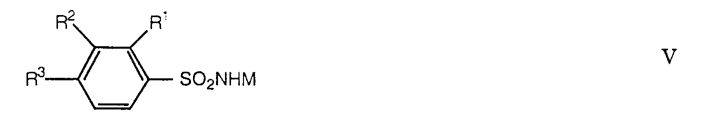 wobei M in Formel V ein Kation darstellt,
zu einer Verbindung der Formel I, worin Y ein Rest R¹⁰ ist und R¹⁻⁸ und X die in Anspruch 1 genannte Bedeutung haben, umsetzt, gegebenenfalls die erhaltene Verbindung der Formel I, in der Y ein Rest CH₃ ist, zu einer Verbindung der Formel I, in der Y ein Rest CHO ist, oxidiert und gegebenenfalls den Rest -CHO in einen anderen, in Anspruch 1 definierten Rest Y überführt.

## Claims

1. Compounds of the formula 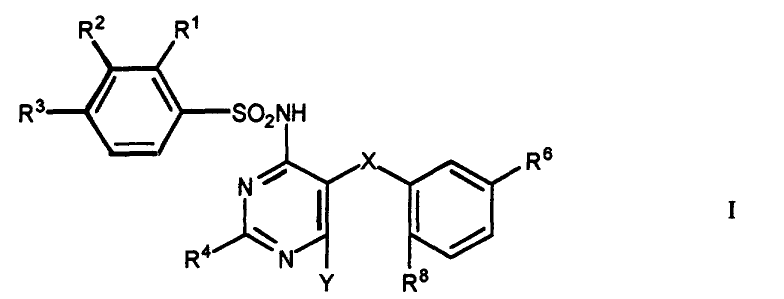 wherein
R¹ represents hydrogen, C₁₋₇-alkyl, C₁₋₇-alkoxy, C₁₋₇-alkylthio, halogen or trifluoromethyl;
R² represents hydrogen, C₁₋₇-alkyl, C₁₋₇-alkoxy, halogen, trifluoromethyl or -OCH₂COOR⁹;
R³ represents hydrogen, C₁₋₇-alkyl; C₁₋₇-alkoxy, halogen, C₁₋₇-alkylthio, trifluoromethyl or trifluoromethoxy;
R² and R³ together represent butadienyl, methylenedioxy, ethylenedioxy or isopropylidenedioxy;
R⁴ represents hydrogen, 2-pyrimidinyl, 2- and 3-furyl, 2- and 3-thienyl, p-methoxyphenyl or morpholino;
R⁶ represents C₁₋₇-alkoxy;
R⁸ represents halogen;
X represents -O- or -S-;
Y represents -CHO, C₁₋₄-alkyl, -(CH₂)₁₋₄-O-R⁹, -(CH₂)₁₋₄NHC(O)R¹⁰, -CH₂OCH₂CH(OH)CH₂OH and cyclic ketals thereof, -CH₂NR⁹CH₂CH(OH)CH₂OH, -CH₂OCH₂CH₂S(O)CH₃, -CH₂O(CH₂)₁₋₄-OH, -CH₂O(CH₂)₁₋₄OC(O)R¹⁰, -CH₂O(CH₂)₁₋₄OC(O)Het, or -CH₂Hal;
R⁹ represents hydrogen or C₁₋₄-alkyl;
R¹⁰ represents C₁₋₄-alkyl;
Het or heterocyclyl represents a mono- or bicyclic 5- or 6-membered heterocycle which can be unsubstituted or mono- or disubstituted with Hal, C₁₋₇-alkyl, C₁₋₇-alkoxy, aryl or aryl-C₁₋₇-alkyl and contains oxygen, nitrogen or sulphur as the hetero atom;
aryl represents phenyl or phenyl substituted with halogen, C₁₋₇-alkyl, C₁₋₇-alkoxy, carboxyl and/or trifluoromethyl; and
Hal is halogen;
and salts thereof.

2. Compounds according to claim 1, wherein Y signifies hydroxymethyl, 2-hydroxyethoxymethyl or 2,3-dihydroxypropoxymethyl.

3. Compounds according to claims 1-2, wherein R¹ and R² signify hydrogen and R³ signifies C₁₋₇-alkyl.

4. The compounds according to claim 1,
4-tert-butyl-N-[5-(2-chloro-5-methoxy-phenoxy)-6-methyl-2-(morpholin-4-yl)-pyrimidin-4-yl]-benzenesulphonamide,
4-tert-butyl-N-[5-(2-chloro-5-methoxy-phenoxy)-6-formyl-2-(morpholin-4-yl)-pyrimidin-4-yl]-benzenesulphonamide,
4-tert-butyl-N-[5-(2-chloro-5-methoxy-phenoxy)-6-hydroxymethyl-2-(morpholin-4-yl)-pyrimidin-4-yl]-benzenesulphonamide,
4-tert-butyl-N-[5-(2-chloro-5-methoxy-phenoxy)-6-chloromethyl-2-(morpholin-4-yl) -pyrimidin-4-yl]-benzenesulphonamide,
4-tert-butyl-N-[5-(2-chloro-5-methoxy-phenoxy)-6-(2-hydroxy-ethoxymethyl)-2-(morpholin-4-yl)-pyrimidin-4-yl]-benzenesulphonamide,
4-tert-butyl-N-[5-(2-chloro-5-methoxy-phenoxy)-6-methyl-pyrimidin-4-yl]-benzenesulphonamide,
4-tert-butyl-N-[5-(2-chloro-5-methoxy-phenoxy)-6-formyl-pyrimidin-4-yl]-benzenesulphonamide,
4-tert-butyl-N-[5-(2-chloro-5-methoxy-phenoxy)6-hydroxy-methyl-pyrimidin-4-yl]-benzenesulphonamide,
4-tert-butyl-N-[6-chloromethyl-5-(2-chloro-5-methoxy-phenoxy)-pyrimidin-4-yl]-benzenesulphonamide,
4-tert-butyl-N-[5-(2-chloro-5-methoxy-phenoxy)-6-(2-hydroxy-ethoxymethyl)-pyrimidin-4-yl]-benzenesulphonamide,
thiophene-3-carboxylic acrid 2-[6-(4-tert-butyl-phenylsulphonamino)-5-(2-chloro-5-methoxy-phenoxy)-2-(morpholin-4-yl)-pyrimidin-4-yl-methoxy]-ethyl ester,
(RS)-4-tert-butyl-N-[5-(2-chloro-5-methoxy-phenoxy)-6-(2,2-dimethyl-1,3-dioxolan-4-ylmethoxymethyl)-2-morpholin-4-yl)-pyrimidin-4-yl]-benzenesulphonamide,
(R,S)-4-tert-butyl-N-[5-(2-chloro-5-methoxy-phenoxy)-6-(2,3-dihydroxypropoxymethyl)-2-(morpholin-4-yl)-pyrimidin-4-yl]-benzenesulphonamide,
4-tert-butyl-N-[5-(2-chloro-5-methoxy-phenoxy)-6-(3-hydroxy-propoxymethyl)-pyrimidin-4-yl]-benzenesulphonamide,
(RS)-4-tert-butyl-N-[5-(2-chloro-5-methoxy-phenoxy)-6-(2,3-dihydroxypropylaminomethyl) -pyrimidin-4-yl]-benzenesulphonamide,
(RS)-4-tert-butyl-N-[5-(2-chloro-5-methoxy-phenoxy)-6-(2,2-dimethyl-1,3-dioxolan-4-ylmethoxymethyl)-pyrimidin-4-yl]-benzenesulphonamide,
(RS)-4-tert-butyl-N-[5-(2-chloro-5-methoxy-phenoxy)-6-(2,3-dihydroxypropoxymethyl)-pyrimidin-4-yl]-benzenesulphonamide,
pyridin-3-ylacetic acid 2-[6-(4-tert-butyl-phenylsulphonylamino)-5-(2-chloro-5-methoxy-phenoxy)-pyrimidin-4-ylmethoxy]-ethyl ester,
pyridin-4-ylacetic acid 2-[6-(4-tert-butyl-phenylsulphonylamino)-5-(2-chloro-5-methoxy-phenoxy)-pyrimidin-4-ylmethoxy]-ethyl ester,
furan-3-carboxylic acid 2-[6-(4-tert-butyl-phenylsulphonylamino)-5-(2-chloro-5-methoxy-phenoxy)-pyrimidin-4-ylmethoxy]-ethyl ester,
thiophene-3-carboxylic 2-[6-(4-tert-butyl-phenylsulphonylamino)-5-(2-chloro-5-methoxy-phenoxy)-pyrimidin-4-ylmethoxy]-ethyl ester and
4-tert-butyl-N-[5-(2-chloro-5-methoxy-phenoxy)-6-methyl-2,2'-bipyrimidinyl-4-yl]-benzenesulphonamide,
(S)-4-tert.-butyl-N-[5-(2-chloro-5-methoxy-phenoxy)-6-(2,2-dimethyl-1,3-dioxolan-4-ylmethoxymethyl) -2-morpholin-4-yl)-pyrimidin-4-yl]-benzenesulphonamide,
(R)-4-tert.-butyl-N-[5-(2-chloro-5-methoxy-phenoxy)-6-(2,2-dimethyl-1,3-dioxolan-4-ylmethoxymethyl)-2-(morpholin-4-yl)-pyrimidin-4-yl]-benzenesulphonamide,
(S)-4-tert.-butyl-N-[5-(2-chloro-4-methoxy-phenoxy)-6-(2,3-dihydroxypropoxymethyl)-2-(morpholin-4-yl)-pyrimidin-4-yl]-benzenesulphonamide,
(R)-4-tert.-butyl-N-[5-(2-chloro-5-methoxy-phenoxy)-6-(2,3-dihydroxypropoxymethyl)-2-(morpholin-4-yl)-pyrimidin-4-yl-benzenesulphonamide,
6-(4-tert.-butyl-phenylsulphonamino)-5-(2-chloro-5-methoxy-phenoxy)-2,2'-bipyrimidinyl-4-carboxaldehyde,
4-tert.-butyl-N-[5-(2-chloro-5-methoxy-phenoxy)-6-hydroxymethyl-2,2'-bipyrimidin-4-yl]-benzenesulphonamide,
4-tert.-butyl-N-[5-(2-chloro-5-methoxy-phenoxy) -6-chloromethyl-2,2'-bipyrimidin-4-yl]-benzenesulphonamide and
4-tert.-butyl-N-[5-(2-chloro-5-methoxy-phenoxy) -6-(2-hydroxyethoxymethyl)-2-2'-bipyrimidin-4-yl]-benzenesulphonamide.

5. The compounds according to any one of claims 1-4 for use as medicaments.

6. The use of the compounds of claims 1-4 for the production of medicaments for the treatment of disorders which are associated with endothelin activities, especially circulatory disorders such as hypertension, ischaemia, vasospasms and angina pectoris.

7. Pharmaceutical preparations in unit dosage form containing a compound of claims 1-4 and a conventional pharmaceutical carrier material.

8. A process for the manufacture of the compounds of claims 1-4, **characterized by** reacting a compound of the formula 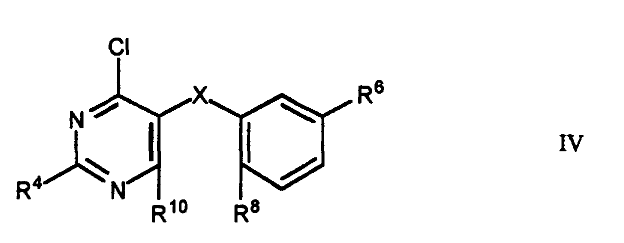 wherein R⁴-R⁸, X and n have the significances set forth in claim 1 and R¹⁰ is C₁₋₄-alkyl,
with a compound of the formula 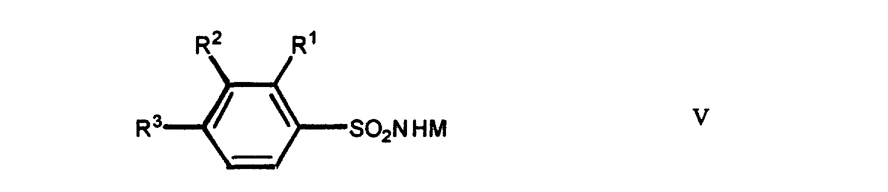 wherein M in formula V represents a cation,
to give a compound of formula I in which Y is a residue R¹⁰ and R¹⁻⁸ and X have the significance set forth in claim 1, if desired, oxidizing the compound of formula I obtained in which Y is a residue CH₃ to a compound of formula I in which Y is a residue CHO and, if desired, converting the residue -CHO into a different residue Y defined in claim 1.

## Revendications

1. Composés de formule 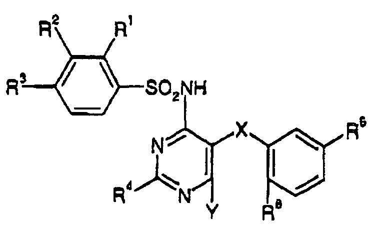 dans laquelle
R¹ représente un atome d'hydrogène ou d'halogène ou un groupe alkyle en C₁₋₇, alcoxy en C₁₋₇, alkyl(C₁₋₇)thio ou trifluorométhyle ;
R² représente un atome d'hydrogène ou d'halogène ou un groupe alkyle en C₁₋₇, alcoxy en C₁₋₇, trlfluorométhyle ou -OCH₂COOR⁹;
R³ représente un atome d'hydrogène ou d'halogène ou un groupe alkyle en C₁₋₇, alcoxy en C₁₋₇, alkyl(C₁₋₇)thio, trifluorométhyle ou trifluorométhoxy ;
R² et R³ peuvent former ensemble un groupe butadiényle, méthylènedioxy, éthylènedioxy ou isopropylidènedioxy ;
R⁴ représente un atome d'hydrogène ou le groupe 2-pyrimidinyle, 2- ou 3-furyle, 2- ou 3-thiényle, p-méthoxyphényle ou morpholino ;
R⁶ représente un groupe alcoxy en C₁₋₇ ;
R⁸ représente un atome d'halogène ;
X représente -O- ou -S- ;
Y représente un groupe -CHO, alkyle en C₁₋₄, -(CH₂)₁₋₄-O-R⁹, -(CH₂)₁₋₄NHC(O)R¹⁰, -CH₂OCH₂CH(OH)CH₂OH et leur cétals cycliques, -CH₂NR⁹CH₂CH(OH)CH₂OH, -CH₂OCH₂CH₂S(O)CH₃, -CH₂O(CH₂)₁₋₄-OH, -CH₂O(CH₂)₁₋₄OC(O)R¹⁰, -CH₂O(CH₂)₁₋₄-OC(O)Het ou -CH₂Hal;
R⁹ représente un atome d'hydrogène ou un groupe alkyle en C₁₋₄;
R¹⁰ représente un groupe alkyle en C₁₋₄;
Het ou hétérocyclyle représente un hétérocycle mono- ou bicyclique à 5 ou 6 chaînons, qui peut être non substitué ou mono- ou disubstitué par un ou des atomes d'halogène ou groupes alkyle en C₁₋₇, alcoxy en C₁₋₇, aryle ou aryl-alkyle(C₁₋₇) et contient un atome d'oxygène, d'azote ou de soufre en tant qu'hétéroatome ;
aryle représente le groupe phényle ou un groupe phényle substitué par un atome d'halogène ou par un groupe alkyle en C₁₋₇, alcoxy en C₁₋₇, carboxy et/ou trifluorométhyle, et
Hal est un atome d'halogène ;
et sels de tels composés.

2. Composés selon la revendication 1, dans lesquels Y représente le groupe hydroxyméthyle, 2-hydroxyéthoxyméthyle ou 2,3-dihydroxypropoxyméthyle.

3. Composés selon les revendications 1 et 2, dans lesquels R¹ et R² représentent des atomes d'hydrogène et R³ représente un groupe alkyle en C₁₋₇.

4. Composés selon la revendication 1,
4-tert-butyl-N-[5-(2-chloro-5-méthoxyphénoxy)-6-méthyl-2-(morpholin-4-yl)-pyrimidin-4-yl]benzènesulfonamide,
4-tert-butyl-N-[5-(2-chloro-5-méthoxyphénoxy)-6-formyl-2-(morpholin-4-yl)-pyrimidin-4-yl]benzènesulfonamide,
4-tert-butyl-N-[5-(2-chloro-5-méthoxyphénoxy)-6-hydroxyméthyl-2-(morpholin-4-yl)pyrlmidin-4-yl]benzènesulfonamide,
4-tert-butyl-N-[5-(2-chloro-5-méthoxyphénoxy)-6-chlorométhyl-2-(morpholin-4-yl)pyrimidin-4-yl]benzènesulfonamide,
4-tert-butyl-N-[5-(2-chloro-5-méthoxyphénoxy)-6-(2-hydroxyéthoxyméthyl-2-(morpholin-4-yl)pyrimidin-4-yl]benzènesulfonamide,
4-tert-butyl-N-[5-(2-chloro-5-méthoxyphénoxy)-6-méthylpyrimidin-4-yl]benzènesulfonamide,
4-tert-butyl-N-[5-(2-chloro-5-méthoxyphénoxy)-6-formylpyrimidin-4-yl]benzènesulfonamide,
4-tert-butyl-N-[5-(2-chloro-5-méthoxyphénoxy)-6-hydroxyméthylpyrimidin-4-yl]-benzènesulfonamide,
4-tert-butyl-N-[6-chlorométhyl-5-(2-chloro-5-méthoxyphénoxy)pyrimidin-4-yl]-benzènesulfonamide,
4-tert-butyl-N-[5-(2-chloro-5-méthoxyphénoxy)-6-(2-hydroxyéthoxyméthyl)-pyrimidin-4-yl]benzènesulfonamide,
thiophène-3-carboxylate de 2-[6-(4-tert-butylphénylsulfonamino)-5-(2-chloro-5-méthoxyphénoxy)-2-(morpholin-4-yl)pyrimidin-4-ylméthoxy]éthyle,
(RS)-4-tert-butyl-N-[5-(2-chloro-5-méthoxyphénoxy)-6-(2,2-diméthyl-1,3-dioxolann-4-ylméthoxyméthyl)-2-(morpholin-4-yl)pyrimidin-4-yl]benzènesulfonamide,
(R,S)-4-tert-butyl-N-[5-(2-chloro-5-méthoxyphénoxy)-6-(2,3-dihydroxypropoxyméthyl)-2-(morpholin-4-yl)pyrimidin-4-yl]benzènesulfonamide,
4-tert-butyl-N-[5-(2-chloro-5-méthoxyphénoxy)-6-(3-hydroxypropoxyméthyl)-pyrimidin-4-yl]benzènesulfonamide,
(R,S)-4-tert-butyl-N-(5-(2-chloro-5-méthoxyphénoxy)-6-(2,3-dihydroxypropylaminométhyl)pyrimidin-4-yl]benzènesulfonamide,
(R,S)-4-tert-butyl-N-[5-(2-chloro-5-méthoxyphénoxy)-6-(2,2-diméthyl-1,3-dioxolann-4-ylméthoxyméthyl)pyrimidin-4-yl]benzènesulfonamide,
(R,S)-4-tert-butyl-N-[5-(2-chloro-5-méthoxyphénoxy)-6-(2,3-dihydroxypropoxyméthyl)pyrimidin-4-yl]benzènesulfonamide,
pyridin-3-ylacétate de 2-[6-(4-tert-butylphénylsulfonylamino)-5-(2-chloro-5-méthoxyphénoxy)pyrimidin-4-ylméthoxy]éthyle,
pyridin-4-ylacétate de 2-[6-(4-tert-butylphénylsulfonylamino)-5-(2-chloro-5-méthoxyphénoxy)pyrimidin-4-ylméthoxy]éthyle,
furann-3-carboxylate de 2-[6-(4-tert-butylphénylsulfonylamino)-5-(2-chloro-5-méthoxyphénoxy)pyrimidin-4-ylméthoxy]éthyle,
thiophène-3-carboxylate de 2-[6-(4-tert-butylphénylsulfonylamino)-5-(2-chloro-5-méthoxyphénoxy)pyrlmidin-4-ylméthoxy)éthyle et
4-tert-butyl-N-[5-(2-chloro-5-méthoxyphénoxy)-6-méthyl-2,2'-bipyrimidinyl-4-yl]benzènesulfonamide,
(S)-4-tert-butyl-N-[5-(2-chloro-5-méthoxyphénoxy)-6-(2,2-diméthyl-1,3-dloxolann-4-ylméthoxyméthyl)-2-(morphotin-4-yl)pyrimidin-4-yl]benzènesulfonamide,
(R)-4-tert-butyl-N-[5-(2-chloro-5-méthoxyphénoxy)-6-(2,2-diméthyl-1,3-dioxolann-4-ylméthoxyméthyl)-2-(morpholin-4-yl)pyrimidin-4-yl]benzènesulfonamide,
(S)-4-tert-butyl-N-[5-(2-chloro-5-méthoxyphénoxy)-6-(2,3-dihydroxypropoxyméthyl)-2-(morpholin-4-yl)pyrimidin-4-yl]benzènesulfonamide,
(R)-4-tert-butyl-N-[5-(2-chloro-5-méthoxyphénoxy)-6-(2,3-dlhydroxypropoxyméthyl)-2-(morpholin-4-yl)pyrimidin-4-yl]benzènesulfonamide,
6-(4-tert-butylphénylsulfonamino)-5-(2-chloro-5-méthoxyphénoxy)-2,2'-bipyrimidinyl-4-carboxaldéhyde,
4-tert-butyl-N-[5-(2-chloro-5-méthoxyphénoxy)-6-hydroxyméthyl-2,2'-bipyrimidin-4-yl]benzènesulfonamide,
4-tert-butyl-N-[5-(2-chloro-5-méthoxyphénoxy)-6-chlorométhyl-2,2'-bipyrimidin-4-yl]benzènesulfonamide et
4-tert-butyl-N-[5-(2-chloro-5-méthoxyphénoxy)-6-(2-hydroxyéthoxyméthyl)-2,2'-bipyrimidin-4-yl]benzènesulfonamide.

5. Composés selon les revendications 1-4, pour utilisation en tant que médicaments.

6. Utilisation des composés des revendications 1-4 dans la fabrication de médicaments destinés au traitement de maladies qui sont associées aux activités de l'endothéline, en particulier de maladies circulatoires telles que l'hypertonie, l'ischémie, les angiospasmes et l'angine de poitrine.

7. Préparations pharmaceutiques sous forme de doses unitaires, contenant un composé des revendications 1-4 et un véhicule pharmaceutiquement usuel.

8. Procédé pour la préparation des composés des revendications 1-4,
**caractérisé en ce qu'**on fait réagir un composé de formule 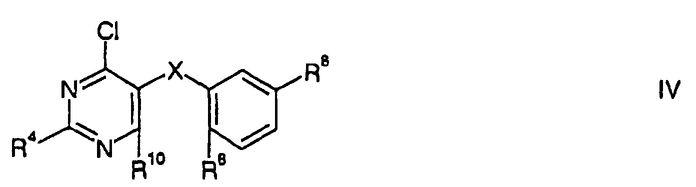 dans laquelle R⁴-R⁸ et X ont les significations données dans la revendication 1 et R¹⁰ est un groupe alkyle en C₁₋₄,
avec un composé de formule 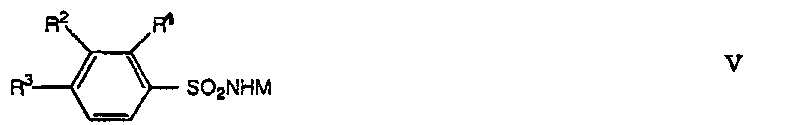 M dans la formule V représentant un cation,
pour aboutir à un composé de formule I, dans lequel Y est un radical R¹⁰ et R¹-R⁶ et X ont les significations données dans la revendication 1, le composé obtenu de formule I, dans lequel Y est un radical CH₃, est éventuellement oxydé en un composé de formule I dans lequel Y est un radical CHO, et éventuellement on convertit le radical -CHO en un autre radical Y défini dans la revendiration 1.
